# EUROPEAN PATENT APPLICATION

(11) **EP 1 886 687 A1**
(43) Date of publication of application: **13.02.2008**
(21) Application number: 06450111.7
(22) Date of filing: 07.08.2006
(51) Int. Cl.: A61K 31/663, A61K 31/675, A61P 19/10

(54) **Intermittent treatment with bone anti-resorptive compounds**

(71) Applicant: Ludwig-Maximilians-Universität, 80539 München (DE)
(72) Inventor: Reinhold, Erben, 2531 Gaaden (AT)
(74) Representative: Sonn & Partner Patentanwälte

(57) **Abstract**

The present invention provides the use of a bone antiresorptive compound for the manufacture of a pharmaceutical composition for a bone metabolism marker monitored bone mass augmenting therapy, characterized in that the bone mass augmenting therapy is a bone anabolic therapy, which is defined by an increase of mineralized bone volume, and the pharmaceutical composition is for intermittent administration starting with an administration of the pharmaceutical composition and at least one successive administration, wherein the successive administration is performed after an increase of the bone metabolism marker by at least 10%.

## Description

The present invention relates to the field of bone mass augmenting therapy.

One of the greatest challenges in the therapy of osteoporosis is to find a cost-effective and safe bone anabolic treatment that would effectively increase bone tissue in an osteopenic skeleton. The major substance classes that have been shown to possess bone anabolic properties *in vivo* are intermittent parathyroid hormone (PTH), prostaglandins, fibroblast growth factors, growth hormone, and vitamin D analogs. Although fluoride salts increase bone mineral density (BMD) in osteoporotic patients, this compound class lacks anti-fracture efficacy.

Newly developed bisphosphonates such as risedronate and alendronate are potent inhibitors of the bone resorptive capacity of mature osteoclasts, and may also induce osteoclast apoptosis. Bisphosphonates prevent estrogen deficiency-induced bone loss in animals, and these drugs are also very effective in the prevention and treatment of postmenopausal osteoporosis. However, although bisphosphonates increase BMD at several bone sites, and significantly reduce spine and hip fracture rates, they do not increase cancellous bone area measured by histomorphometry in osteoporotic patients (1). Therefore, continuous treatment with these drugs is an antiresorptive therapy, but cannot be considered to be bone anabolic. So far, treatment of osteoporotic patients with bisphosphonates has mostly been conducted as a continuous therapy with daily or weekly administration of the drug (reviewed in (2)).

Besides their clear antiresorptive activity, it has been suggested that bisphosphonates inhibit glucocorticoid-induced osteoblast and osteocyte apoptosis *in vivo* in mice. Increased cell survival under the influence of bisphosphonates may enhance the performance of individual osteoblast teams. In line with the idea that bisphosphonates may inhibit osteoblast apoptosis, it has been reported that wall thickness and remodeling balance may increase under chronic treatment with risedronate in dogs. "Wall thickness" is the mean thickness of completed remodeling units. This value reflects the amount of newly formed bone during the formation phase of the bone remodeling cycle, and, therefore, is a measure of the performance of individual osteoblast teams (3). In contrast, a 3-year study in dogs treated with high chronic doses of alendronate did not reveal any changes in cancellous bone volume or architecture (4). In addition, cancellous bone volume and wall thickness were unchanged after 1 year of alendronate treatment in patients with glucocorticoid-induced osteoporosis (1). In postmenopausal osteoporotic women an increase in wall thickness was observed after 2 years, but not after 3 years of alendronate treatment. Cancellous bone volume remained unchanged in the latter study.

It has been clearly shown that continuous administration of risedronate to rats dose-dependently suppressed osteoblast perimeter, mineralizing perimeter, mineral apposition rate and wall thickness, clearly indicating that risedronate decreased osteoblast recruitment and also osteoblast team performance in OVX rats (5). Therefore, the available experimental and human data do not provide evidence to support the hypothesis that continuous treatment with bisphosphonates has a positive effect on wall thickness and osteoblast team performance. In contrast, there is firm evidence that continuous treatment with bisphosphonates is not bone anabolic, because this treatment does not increase cancellous bone mass measured, e.g. by bone histomorphometry in humans (1) or animals (4,5).

It is therefore a goal of the present invention to provide means for a bone anabolic therapy, which increases the volume of cancellous bone and bone wall thickness.

Therefore the present invention provides the use of a bone antiresorptive compound, preferably a bisphosphonate compound, for the manufacture of a pharmaceutical composition for a bone metabolism marker monitored bone mass augmenting therapy, characterized in that the bone mass augmenting therapy is a bone anabolic therapy, which is defined by an increase of mineralized bone volume, preferably in cancellous bone or by bone wall thickness, and the pharmaceutical compound is for intermittent or intermitted administration starting with an administration of the pharmaceutical composition and at least one successive administration, wherein the successive administration is performed after an increase of the bone metabolism marker by at least 10%.

Preferred bone antiresorptive compounds (also referred to as "antiresorptives"), especially potent antiresorptive compounds, are bisphosphonate compounds or compounds with bisphosphonate-like activity.

It was surprising that a one time administration, e.g. a single injection, of a bisphosphonate like risedronate increases wall thickness of completed bone remodeling units several months post treatment in aged female retired breeder rats. Therefore, repeated treatment with single doses of a bone antiresorptive compound can be exploited as a bone anabolic treatment. Single doses of bone antiresorptive compounds are administered repeatedly, but only during the high turnover phases when bone turnover recovers, long after the preceding administration of a antiresorptive (bisphosphonate or other equivalent bone regulating agents having bisphosphonate-like activity). Recently, it was discovered that special antibodies against RANKL (Bekker PJ et al., J Bone Miner Res 19, (2004):1059-66, McClung et al., N Engl J Med 23 (2006): 821-31) or recombinant OPG (Caparelli et al., J Bone Miner Res 18 (2003):852-8), targeting essential signal factors for osteoclast recruitment and osteoclast activity, also have strong antiresorptive effects on bone. The suppression of bone turn-over is in the range of the most potent bisphosphonates. A RANKL antibody or recombinant OPG are also equivalent bone regulating agents, which can be used instead or in addition of a bisphosphonate. The inventive bone anabolic efficacy is based on a positive remodeling balance (visible through the increase in wall thickness) and on the induction of modeling drifts (1). Therefore in a specific embodiment of the present invention the bone antiresorptive compound is an antibody specific for RANKL or recombinant OPG.

Apparently a new mechanism underlies this form of intermittent treatment. Continuous treatment with antiresorptives has the opposite effect in rats, i.e., wall thickness declines (5). Although antiresorptives, especially highly potent bisphosphonates such as ibandronate or zoledronate, have been given intermittently with between-dose intervals longer than 1 week, the rationale for intermittent therapy has been the reduction of untoward side effects and the improvement of adherence to therapy (2). A previously known intermittent bisphosphonate therapy is considered an antiresorptive therapy (2), wherein the bisphosphonate is given in constant cycles without different effects on bone growth or bone mass augmentation. Since the long lasting effects based on adsorption to bones bisphosphonate administration in weekly intervals has the same effects as continuous administration beside a greater convenience for the patient and therapy adherence (2). Therefore, the intermittent treatment with antiresorptives known so far is different in application as well as in their effects from the invention described herein.

The critical issue to get a bone anabolic effect for intermittent therapy with antiresorptives is that the drugs are always given during a phase when bone turnover is high or at least increasing. Without being limited to a certain theory - because the effect of antiresorptives on bone resorption is long-lasting due to the mode of their action (6), bone resorption is more inhibited than bone formation during the weeks and months after the injection, resulting in a positive bone balance. In addition, it is conceivable that the injection of a powerful antiresorptive drug such as a bisphosphonate will induce an increase in parathyroid hormone (PTH) secretion to counteract the reduced flow of calcium from bone, especially in states of high bone turnover. In the presence of the inhibitory effect of antiresorptives on bone resorption, increased PTH may further contribute to a positive bone remodeling balance by its stimulatory action on bone formation. In addition, this putative PTH surge may induce modeling drifts on resting bone surfaces. According to the present invention the timing of the treatments with the antiresorptive is performed according to the actual effect of the drug on bone turnover in individual patients. For example, the next treatment is initiated when bone markers indicate a recovery of bone turnover. Such a treatment regimen makes it possible to conduct an individualized bone anabolic therapy. After several injections/treatments, bone mass may increase, especially in patients with high bone turnover.

Taken together, intermittent therapy with single doses of antiresorptives may be a safe and cost-effective bone anabolic treatment with a greatly reduced amount of required treatment agents.

The present invention also relates to the method of a bone mass augmenting therapy, characterized in that the bone mass augmenting therapy is a bone anabolic therapy, which is defined by an increase of mineralized bone volume, and the pharmaceutical compound is for intermittent administration starting with an administration of the pharmaceutical composition and at least one successive administration, wherein the successive administration is performed after an increase of a monitored bone metabolism marker by at least 10%. In this regard an effective amount of the antiresorptive compound is administered, wherein the amount can be determined by efficiency, pharmacodynamic factors and side-effects of the antiresorptive compound. These factors are already generally known for antiresorptives such as bisphosphonates, even for single administrations in given intervals.

The bone mass augmenting therapy is for example a therapy of osteoporosis.

In particular the present invention utilizes monitoring of known bone metabolism markers. Such a marker is for example serum osteocalcin or urinary DPD (deoxypyridinoline). After the administration of the antiresorptive these markers generally decrease (relative to the value of a sample taken prior to the administration). After a certain monitoring time span the marker values eventually increase again. After a certain increase, e.g. of preferably +10% relative to the lowest value since the previous administration an additional administration is performed. This time span varies from organism to organism or individual to individual and can even reach one or two years. During the long time span the increase in bone volume or bone mass augmentation takes effect, without being negatively effected by an additional administration of antiresorptives. In certain embodiments the increase of a bone metabolism marker is by at least 15%, preferably 20% , more preferred by at least 25%, most preferred by at least 30%.

A preferred class of bone metabolism markers are bone resorption markers. Such markers indicate the resorption of bone constituents into the blood and monitor directly the activity of the target bone cells. Such markers are for example fasting urinary hydroxyproline, N-telopeptide of the alpha chain of type I collagen (NTX-I) (e.g. measured in urine), C-telopeptide of the alpha chain of type I collagen (CTX-I) (serum or urine), pyridinoline and deoxypyridinoline (e.g. in urine, generally as pyridinium crosslinks), cathepsine K, tartrate resistant acid phosphatase 5b, bone sialoprotein, osteocalcin fragments, osteopontin, OPG, RANKL, CTX-I isomeres.

In another embodiment the bone metabolism marker is a bone formation marker, usually used to monitor osteoblast activity, such as (bone-specific) alkaline phosphatase, osteocalcin, PINP (procollagen type I N-terminal propeptide) or PICP (procollagen type I C-terminal peptide).

Preferred bone metabolism markers are selected from at least one of osteocalcin, alkaline phosphatase, deoxypyridinoline, hydroxyproline, N-telopeptide of the alpha chain of type I collagen (NTX-I) and C-telopeptide of the alpha chain of type I collagen (CTX-I), preferably deoxypyridinoline, NTX-I or CTX-I. In other embodiments two, three, four or five markers are monitored wherein the relative mean value of the markers is taken into account, or alternatively when one, two, three, four or five markers reach the desired increase threshold (e.g. +10%). Osteocalcin can for example be measured by antibody based assays as disclosed by the EP 0646794.

Preferably the bone metabolism marker is measured relative to creatinine, in particular urine markers such as the markers deoxypyridinoline, hydroxyproline, N-telopeptide of the alpha chain of type I collagen and C-telopeptide of the alpha chain of type I collagen. For better reproducibility and to negate incidental fluctuation this relative value of two measurements is taken into account when monitoring the increase threshold for the next administration.

In particular the bone metabolism marker is measured in urine or blood of a patient, including samples of these bodily fluids.

For full effect of the bone anabolic treatment it is preferred that between a first administration and the increase of the bone metabolism marker no further administration of the antiresorptive compound is performed.

The antiresorptive compound is for example a bisphosphonate selected from alendronate, risedronate, etidronate, ibandronate, clodronate, neridronate, pamidronate or zoledronate, preferably alendronate or risedronate, or a mixture thereof.

Preferably the specific increase of the bone metabolism marker is preceded by a drop of the bone metabolism marker after an administration of antiresorptives. This drop usually occurs immediately after administration and reaches preferably a decrease by at least 30%, preferably by at least 40%, most preferred by at least 50% relative to the bone metabolism marker measurement prior to the first measurement of the bone metabolism marker in a sample taken prior to the first administration. To effect this decrease a sufficient amount of antiresorptive can be administered, which can be calculated taking patient parameters like weight and size, as well as age into account. If necessary a further proximate administration can be performed (which does count together with the initial administration as a primary administration after which the increase will be monitored), e.g. within 12, 24, 36 or 48 weeks (depending on the organism) in humans, to achieve the desired decrease.

For procedural exactness the bone metabolism marker is preferably measured directly (e.g. on the same day or the following day, but at least before the next measurement) before an administration and in intervals of at least 1 week, preferably at least 2 weeks, more preferred at least 1 month, most preferred at least 2 months.

Preferably the administration is in one dose, the antiresorptive compound can however also be administered in consecutive smaller doses if desired within e.g. 1, 2, 3, 4, 5 or 6 days, or 1, 2, 3 or 4 weeks or in special cases up to 12, 36 or 48 weeks.

Preferably the administered dose of the antiresorptive compound, in particular a bisphosphonate, is between 0,5 mg and 200 mg, preferably between 1 mg and 50 mg, most preferred between 2 mg and 10 mg.

Preferably the increase of the bone metabolism marker is relative between two consecutive measurements of the marker during the monitoring period to determine the next administration date.

For greater reliability and to exclude outliers preferably the increase of the bone metabolism marker is relative between the average of two, preferably three or four, consecutive measurements and the average of following two, preferably three or four, consecutive measurements.

The present invention is further illustrated by the following figures and examples without being limited thereto.

### Figures

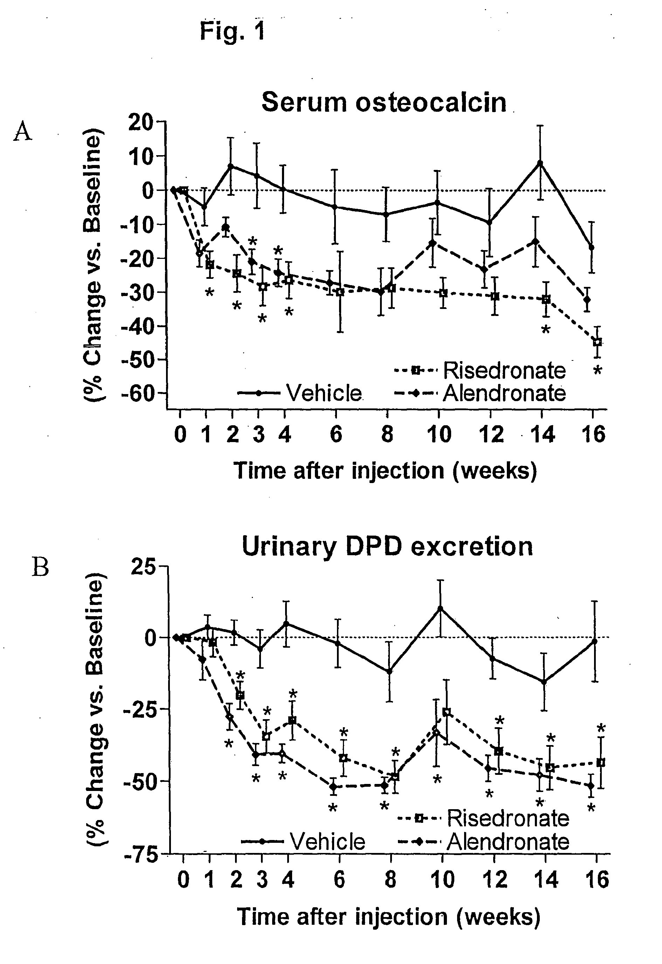

Fig. 1: Development of serum osteocalcin (A) and urinary DPD (deoxypyridinoline) excretion (B) after a single bisphosphonate administration.

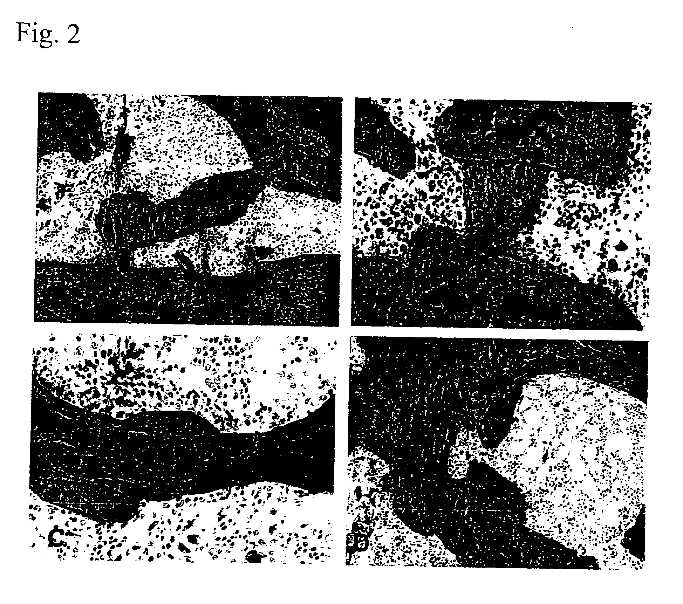

Fig. 2: Bone cross sections showing the formation of modeling drifts in bisphosphonate-treated retired breeder rats. A-C: risedronate-treated, D: alendronate-treated; 5 µm thick undecalcified methylmethacrylate (MMA) sections surface-stained with toluidine blue; magnification A and D: x200, B and C: x400. Bone formation on top of smooth cement lines (A-D, arrows) indicate the induction of modeling drifts in the animals. The modeling drifts can reconnect bone trabeculae by bridging the marrow space between isolated structural elements (in A, B, D).

### Examples

### Example 1: Material and Methods

### Animal Procedures

All animal procedures were conducted in accordance with German Animal Welfare Regulations. Thirty-six female, approximately 6 to 9-months-old, retired breeder Fischer 344 rats (Charles River WIGA, Raleigh, SC, Germany) weighing 180 - 250 g were used for this experiment. The animals were weight-matched into the three groups (12/group) shown in Table 1. The experiment lasted 4 months. At baseline, each rat was injected intravenously via a tail vein with vehicle (physiological saline) or with 50 µg/kg alendronate or risedronate. The injection volume was 1 ml/kg for all rats. In order to assess the time course of the effect of the bisphosphonate injection on bone turnover, serial blood and urine sampling was performed. For the collection of urine, all animals were kept in metabolic cages for 15 hours overnight: a) prior to the start of the experiment; b) at weekly intervals during the first month; and c) at biweekly intervals thereafter (Table 1). On the following morning, a 200 - 300 µl blood sample was collected from a tail vein. Calcein (20 mg/kg) was injected subcutaneously on the 8th and 3rd day before the rats were killed. The animals were housed in pairs at 22° C with a 12 hour light/12 hour dark cycle and were fed a Standard laboratory chow (Altromin 1320, Altromin, Lage, Germany) containing 0.9% calcium, and 0.7% phosphorus. Food and tap water was available ad libitum to all rats. Body weights were taken weekly. At the end of the experiment, all rats were killed by exsanguination from the abdominal aorta under ketamine/xylazine anesthesia, 16 weeks post treatment. Serum samples were stored at -80°C, and urine samples at -35°C until assayed.

**Table 1. Treatment Groups and Timings**

| Treatment | Dose | No | Sample Collections and Timings (Weeks) | | |
|---|---|---|---|---|---|
| | | | Blood and Urine | Calcein Label | Sacrifice |
| Vehicle | - | 12 | 0,1,2,3,4,6,8,10,12,14,16 | 15 and 16 | 16 |
| Risedronate | 50 µq/kg | 12 | 0,1,2,3,4,6,8,10,12,14,16 | 15 and 16 | 16 |
| Alendronate | 50 µg/kg | 12 | 0,1,2,3,4,6,8,10,12,14,16 | 15 and 16 | 16 |

### Serum Analysis

Total calcium, potassium and sodium in serum and urine were determined by flame photometry (EFOX 5053, Eppendorf, Hamburg, Germany). Serum alkaline phosphatase activity, aspartate transaminase, creatine kinase, albumin, total bilirubin, serum urea, creatinine, and phosphorus as well as urinary creatinine and phosphorus were analyzed on a Hitachi 766 Autoanalyzer (Boehringer Mannheim, Mannheim, Germany). Serum osteocalcin was measured by immunoradiometric assay (IRMA) specific for rat osteocalcin (Biomedical Technologies, Staughton, IN). Total deoxypyridinoline concentrations in urine were determined after acid hydrolysis by ELISA (Total DPD, Metra Biosystems, Mountain View, CA).

### Bone mineral density/volume measurements

Bone mineral density and volume via cross section area of the left tibiae and of the L4 vertebrae was measured by peripheral quantitative computed tomography (pQCT) using a XCT Research M+ pQCT machine (Stratec Medizintechnik, Pforzheim, Germany). The measurements were made with a collimator opening of 0.2 mm. In the tibiae, three slices were measured. One slice in the mid-diaphysis of the tibiae located 2 mm proximal to the tibiofibular junction, one slice in the proximal tibial metaphysis located 2 mm distal of the growth plate, and one slice in the distal tibia located 2 mm from the articular cartilage. In the vertebrae, 3 transversal slices were measured in the center of the vertebra and 1.6 mm rostral and caudal of the mid-transversal plane. In each vertebra, the final BMD and geometric values were calculated as the mean value over the 3 slices. A voxel size of 0.100 mm and a threshold of 710 mg/cm³ for calculation of cortical BMD were used.

### Histology

At necropsy, the first lumbar vertebrae and the proximal right tibiae were defleshed and fixed in 70% ethanol at room temperature. After fixation the bones were embedded undecalcified in methylmethacrylate, as described previously (7). Five-pm-thick undecalcified sections were prepared with a HM 360 microtome (Microm, Walldorf, Germany). The sections were sampled in the median plane of the vertebrae and the midsagittal plane of the tibiae, and were stained with von Kossa (8), and toluidine blue at acid pH (9). For demonstration of cement lines the surface staining technique described by Schenk et al. (7) was used.

### Histomorphometry

All measurements were performed on the secondary spongiosa of the proximal tibial metaphysis and of the first lumbar vertebral body, and are presented as two-dimensional histomorphometric terms. Histomorphometric parameters were calculated and expressed according to the recommendations made by the ASBMR nomenclature committee (10).

Structural histomorphometric measurements were made with an automatic image analysis system (VIDAS, C. Zeiss, Germany) connected to a Zeiss Stereo microscope (C. Zeiss) via a TV-camera (Bosch, Germany). All measurements with the automatic image analysis system were performed on sections stained with von Kossa at a magnification of the zoom objective of x2.0. A measuring field (about 13 mm² in the tibiae, and about 5 mm² in the vertebrae) encompassing most of the cancellous bone in the L1 vertebrae and the tibiae was used. The cancellous bone within 0.5 mm from the growth plates was excluded from the measurements in both the tibiae and the vertebrae. All values for individual animals are the mean of two measurements made on two different sections that were generally about 100 µm spaced from each other. The image analysis system automatically determined the measuring area (tissue area, TAr), bone area (BAr), and bone perimeter (BPm). From these data, the structural Parameters bone area (BAr/TAr), bone perimeter (BPm/TAr), trabecular width (TbWi), trabecular number (TbN), and trabecular Separation (TbSp) were calculated.

Cellular, dynamic, and BSU related histomorphometric measurements were made using a semiautomatic system (Videoplan, C. Zeiss) and a Zeiss Axioskop microscope with a drawing attachment. In the cancellous bone of the first lumbar vertebra and the proximal tibial metaphysis about 15-25 fields (4-6 mm²) were evaluated in each section. At least 15 mm of cancellous bone perimeter were sampled in the tibia and the vertebra of each animal. The area within 0.5 mm from the growth plates was excluded from the measurements in tibial and vertebral cancellous bone. Static histomorphometric parameters were measured in sections stained with toluidine blue, and dynamic, fluorochrome-based parameters were measured in unstained sections.

The following primary parameters were determined at x200: bone perimeter, osteoid perimeter, osteoblast perimeter, eroded perimeter, osteoclast perimeter, number of osteoclasts, calcein double-labeled perimeter, and tetracycline-labeled perimeter. Osteoid seams with a width smaller than 2 µm were excluded from the measurements. Osteoblasts were defined as mononuclear, basophilic cells in contact with osteoid. Osteoclasts were defined as large, irregularly shaped cells with a foamy, slightly metachromatic cytoplasm containing 1 or more nuclei, and located within Howship's lacunae. Osteoid width was determined directly at x400, sampling each osteoid seam every 50 µm. Osteoid seams with a width smaller than 2 µm were not included. For the measurement of eroded perimeter, only erosions with a depth of at least 3 Mm were included into the measurements.

The interlabel width between calcein double labels was measured at x400, sampling each double label every 50 µm. The mineral apposition rate (MaR) was given by the arithmetic mean of the width measurements, divided by the interlabel period (5 days). Bone formation rate (BFR/BPm) was calculated by multiplying mineralizing perimeter (double labeled bone perimeter) by the MaR. The wall width of completed cancellous bone structural units was measured at x200. The distance between the reversal line and the bone surface was measured at 4 equidistant points in at least 15 remodeling sites in each animal. Values for MaR, osteoid width, and wall width were not corrected for obliquity of the plane of sectioning.

### Statistical analysis

Statistics were computed using SPSS for Windows 11.5 (SPSS, Chicago, IL). The data were analyzed using one-way analysis of variance (ANOVA). When the analysis of variance performed over all groups indicated a significant (P<0.05) difference among the groups, statistical differences between individual groups were subsequently evaluated using Tukey's multiple comparison test as post hoc test. The data are presented as means ± SD. In Figure 1, data are given as means ± SEM.

### Example 2: Results

### Biochemical parameters of bone turnover.

The longitudinal measurements of urinary DPD as a marker of whole body bone resorption and of serum osteocalcin as a marker of whole body bone formation revealed a pronounced reduction in bone turnover after a single injection of 50 µg/kg risedronate and alendronate. There were no significant differences between risedronate- and alendronate-treated rats at any time point. However, risedronate tended to decrease serum osteocalcin more rapidly and also more lasting than alendronate (Fig. 1). Interestingly, the decline in serum osteocalcin was about a week more rapid than the decline in urinary DPD after injection of the bisphosphonates (Fig. 1). In both risedronate- and alendronate-treated rats, urinary DPD excretion had reached a steady state after about 6 weeks post injection. The reduction in urinary DPD excretion induced by bisphosphonate treatment reached about 50% during this experiment. Surprisingly, urinary DPD excretion was still significantly reduced in risedronate- and alendronate-treated rats at the end of the experiment, i.e., 16 weeks after a single injection of 50 µg/kg risedronate or alendronate.

### Bone densitometry

No significant bisphosphonate-induced changes in BMD or overall bone geometry at the tibiae or the lumbar vertebrae at the end of the experiment was found.

### Histomorphometry

In accordance with the pQCT measurements, cancellous bone area or cancellous bone architecture remained unchanged in bisphosphonate-treated rats at the end of the trial. However, in contrast to the whole body biochemical bone markers, static and dynamic Indexes of bone formation did not show a reduction in bisphosphonate-treated compared with vehicle-treated rats. Although there was a trend for lower eroded perimeter, osteoclast perimeter, and osteoclast numbers in the tibiae and vertebrae of risedronate and, to a lesser degree, of alendronate-treated rats, these effects did not reach statistical significance.

Most interesting was that wall width of completed bone remodeling units was increased in the vertebrae of risedronate-treated rats relative to vehicle-treated rats. A similar trend for increased wall width was seen at the tibiae in risedronate-treated rats, and in tibial and vertebral cancellous bone of alendronate-treated animals. Another interesting observation was that bisphosphonate treatment appeared to induce modeling drifts on completed bone remodeling units in some but not all animals (Fig. 2). In some instances, this activity appeared to result in bridges between individual bone spicules.

### Example 3: Discussion

It is generally believed that the decrease in bone formation induced by bisphosphonate treatment is a consequence of their antiresorptive effect through coupling of bone formation and bone resorption. However, this study suggests that the decline in the bone formation marker serum osteocalcin actually precedes the decline in bone resorption markers in aged retired breeder rats. This finding suggests that bisphosphonates have a direct inhibitory action on osteoblastic bone formation.

Another interesting observation in this study was the discrepancy between biochemical markers and histomorphometric findings at the end of the experiment. The lack of significantly reduced histomorphometric Indexes of bone resorption (osteoclast number, osteoclast perimeter, eroded perimeter) in the bisphosphonate-treated rats despite significantly reduced urinary collagen crosslink excretion may be explained by the fact that bisphosphonates mainly inhibit the resorptive capacity of mature osteoclasts (6), and do not necessarily lower osteoclast numbers. The discrepancy between normal dynamic indexes of bone formation in vertebral and tibial cancellous bone in the presence of significantly reduced serum levels of osteocalcin in risedronate-treated rats at the end of the experiment can be explained by the fact that the absolute serum osteocalcin values did not show significant differences between vehicle- and bisphosphonate-treated animals at any time point of the study. Because of the high variability of serum osteocalcin levels in the retired breeder rats, only the relative differences versus baseline showed statistically significant changes.

Clearly the most important finding in this study was that a single injection of risedronate can increase wall width of completed bone remodeling units several months post treatment. In contrast, a continuous 3-months treatment with risedronate decreased wall width in ovariectomized rats (5). Because the effect of bisphosphonates on bone resorption is long-lasting due to the mode of their action (6), it is possible that bone resorption is more inhibited than bone formation during the weeks and months after the injection, resulting in a positive bone balance. In addition, it is conceivable that the injection of a powerful antiresorptive drug such as a bisphosphonate will induce an increase in parathyroid hormone (PTH) secretion to counteract the reduced flow of calcium from bone, especially in states of high bone turnover. In the presence of the inhibitory effect of bisphosphonates on bone resorption, increased PTH further contributes to a positive bone remodeling balance by its stimulatory action on bone formation. In addition, this putative PTH surge may induce modeling drifts on resting bone surfaces. Therefore, repeated treatment with single doses of a bisphosphonate can be exploited as a bone anabolic treatment especially in patients with high bone turnover. The timing of the treatments with the bisphosphonate can be performed according to the actual effect of the drug on bone turnover in individual patients. For example, the next treatment can be initiated when bone markers indicate a recovery of bone turnover. Such a treatment regimen makes it possible to conduct an individualized bone anabolic therapy. After several injections/treatments, bone mass may increase.

### References

1. Chavassieux et al. 2000. J.Bone Miner.Res. 15:754-762.
2. Miller. 2005. Clin.Ther. 27:361-376.
3. Parfitt et al. 1995. J.Bone Miner.Res. 10:466-473.
4. Balena et al. 1996. J.Pharmacol.Exp.Ther. 276:277-283.
5. Erben et al. 2002. J.Bone Miner.Res. 17:1498-1511.
6. Rodan et al. 1996. J.Clin.Invest. 97:2692-2696.
7. Schenk et al. 1984. Dickson GR, ed Methods of calcified tissue preparation. Amsterdam: Elsevier 1-56.
8. Erben et al. 1990. Scanning Microsc; 4:625-38.
9. Baron et al. 1983. Recker RR, ed Bone Histomorphometry: Tech niques and Interpretation, Boca Raton, FL: CRC Press:13-35.
10. Parfitt et al. 1987. J Bone Miner Res 2:595-610.

## Claims

1. Use of a bone antiresorptive compound for the manufacture of a pharmaceutical composition for a bone metabolism marker monitored bone mass augmenting therapy, **characterized in that** the bone mass augmenting therapy is a bone anabolic therapy, which is defined by an increase of mineralized bone volume, and the pharmaceutical composition is for intermittent administration starting with an administration of the pharmaceutical composition and at least one successive administration, wherein the successive administration is performed after an increase of the bone metabolism marker by at least 10%.

2. Use according to claim 1, **characterized in that** the increase of a bone metabolism marker by at least 15%, preferably 20% , more preferred by at least 25%, most preferred by at least 30%.

3. Use according to claim 1 or 2, **characterized in that** the bone metabolism marker is a bone resorption marker.

4. Use according to any one of claims 1 to 3, **characterized in that** the bone metabolism marker is a bone formation marker.

5. Use according to any one of claims 1 to 4, **characterized in that** the bone metabolism marker is selected from at least one of osteocalcin, bone specific alkaline phosphatase, deoxypyridinoline, Hydroxyproline, N-telopeptide of the alpha chain of type I collagen (NTX-I) and C-telopeptide of the alpha chain of type I collagen (CTX-I), PINP, PICP, preferably deoxypyridinoline, NTX-I or CTX-I.

6. Use according to any one of claims 1 to 5, **characterized in that** the bone metabolism marker is measured relative to creatinine, in particular deoxypyridinoline, Hydroxyproline, N-telopeptide of the alpha chain of type I collagen and C-telopeptide of the alpha chain of type I collagen.

7. Use according to any one of claims 1 to 6, **characterized in that** the bone metabolism marker is measured urine or blood of a patient.

8. Use according to any one of claims 1 to 7, **characterized in that** between the first administration and the increase of the bone metabolism marker no further administration of the pharmaceutical composition is performed.

9. Use according to any one of claims 1 to 8, **characterized in that** the bone antiresorptive compound is a bisphosphonate compound, preferably selected from alendronate, risedronate, etidronate, ibandronate, clodronate, neridronate, pamidronate or zoledronate, preferably alendronate, risedronate or a mixtur thereof.

10. Use according to any one of claims 1 to 8, **characterized in that** the bone antiresorptive compound is an antibody specific for RANKL or recombinant OPG.

11. Use according to any one of claims 1 to 10, **characterized in that** the increase of a bone metabolism marker is preceded by a drop of the bone metabolism marker after an administration of the pharmaceutical .

12. Use according to claim 11, **characterized in that** the drop of the bone metabolism marker is by at least 30%, preferably by at least 40%, most preferred by at least 50% relative to the bone metabolism marker prior to the first measurement of the bone metabolism marker.

13. Use according to any one of claims 1 to 12, **characterized in that** the bone metabolism marker is measured directly before an administration and in intervals of at least 1 week, preferably at least 2 weeks, more preferred at least 1 month, most preferred at least 2 months.

14. Use according to any one of claims 1 to 13, **characterized in that** an administration is in one dose.

15. Use according to any one of claims 1 to 14, **characterized in that** a administered dose of the antiresorptive compound, preferably being a bisphosphonate compound, is between 0,5 mg and 200 mg, preferably between 1 mg and 50 mg, most preferred between 2 mg and 10 mg.

16. Use according to any one of claims 1 to 15, **characterized in that** the increase of the bone metabolism marker is relative between two consecutive measurements.

17. Use according to any one of claims 1 to 15, **characterized in that** the increase of the bone metabolism marker is relative between the average of two, preferably three, consecutive measurements and the average of following two, preferably three, consecutive measurements.
